Europäisches Patentamt

European Patent Office

Office européen des brevets

(11) Veröffentlichungsnummer: **0 502 384 B1**

## EUROPÄISCHE PATENTSCHRIFT

(45) Veröffentlichungstag der Patentschrift: **25.01.95**

(21) Anmeldenummer: **92102980.7**

(22) Anmeldetag: **21.02.92**

(51) Int. Cl.⁶: **C07C 51/43**, C07C 55/14

(54) **Verfahren zur Rückgewinnung von Adipinsäure.**

(30) Priorität: **05.03.91 DE 4106937**

(43) Veröffentlichungstag der Anmeldung:
**09.09.92 Patentblatt 92/37**

(45) Bekanntmachung des Hinweises auf die
Patenterteilung:
**25.01.95 Patentblatt 95/04**

(84) Benannte Vertragsstaaten:
**BE DE ES FR GB IT NL**

(56) Entgegenhaltungen:
**FR-A- 1 208 145**
**GB-A- 745 063**
**US-A- 2 713 067**
**US-A- 3 476 805**

(73) Patentinhaber: **BAYER AG**

**D-51368 Leverkusen (DE)**

(72) Erfinder: **Vogel, Peter, Dr.**
**Walter-Flex-Strasse 18**
**D-5090 Leverkusen 1 (DE)**
Erfinder: **Steinhoff, Georg, Dr.**
**Buschstrasse 169**
**D-4150 Krefeld 1 (DE)**

**Beschreibung**

Die Erfindung betrifft ein neues Verfahren zur Rückgewinnung von Adipinsäure aus dem Nebenproduktausgrenzstrom, der bei der Oxidation technischer Gemische von Cyclohexanol/Cyclohexanon mittels Salpetersäure und Isolierung der Hauptmenge der gebildeten Adipinsäure durch Kristallisation anfällt, wobei gleichzeitig der bei der destillativen Aufarbeitung der in der genannten Mutterlauge verbleibenden Glutarsäure anfallende Destillationsrückstand einer sinnvollen Nutzung zugeführt wird.

Bei der Herstellung von Adipinsäure durch Oxidation technischer Gemische von Cyclohexanol/-on mittels Salpetersäure fallen als Nebenprodukte Glutar- und Bernsteinsäure an. Dies sind, relativ zu Adipinsäure, in der Mutterlauge der Adipinsäurekristallisation am stärksten angereichert. Ein Teil dieser Mutterlauge wird daher aus dem Prozeß ausgeschleust, um die Anreicherung der Nebenprodukte zu verhindern. Nach Abtrennung von Salpetersäure und Wasser kann das erhaltene Dicarbonsäuregemisch destillativ zu "Glutarsäure techn." aufgearbeitet werden. Der dabei anfallende Destillationsrückstand enthält überwiegend Adipinsäure, Verkokungsprodukte sowie metallische Katalysatoren, wie z.B. Kupfer oder Vanadin.

Die in dem Dicarbonsäuregemisch enthaltene Adipinsäure (ca 25 bis 35 Gew.-%) geht als Adipinsäure verloren, sie verschlechtert die Qualität der erhaltenen technischen Glutarsäure, beeinträchtigt somit ihre Verwendbarkeit im Gerbstoffbereich und erhöht die Rückstandsmenge bei der destillativen Glutarsäure-Aufarbeitung.

Zur Rückgewinnung von Adipinsäure wurden daher eine Reihe von Verfahren entwickelt. Nach US-Patent 3 790 626 läßt sich Adipinsäure, z.B. mit Cyclohexanol und/oder Cyclohexanon extrahieren. Nach US-Patent 4 146 730 formen Glutar- und Bernsteinsäure mit Harnstoff Addukte, von denen Adipinsäure separiert werden kann.

Ebenso ist die Umwandlung von Glutar- und Bernsteinsäure in Imide und deren Abtrennung beschrieben (US-Patent 3 818 081). Die Reaktion mit Alkylamin verwandelt Glutar- und Bernsteinsäure in Amide, die von Adipinsäure separiert werden (EP-A 33 851). US-Patent 4 442 303 beschreibt die Veresterung der Dicarbonsäuren und die Aufarbeitung und Trennung der Ester.

Auch verschiedene Kristallisationsverfahren sind in der Literatur beschrieben. US-Patent 4 014 903 beschreibt eine einfache Kühlkristallisation zur Rückgewinnung von Adipinsäure aus dem Nebenproduktausgrenzstrom. Die Effizienz eines solchen Verfahrens bleibt jedoch, wie Versuche zeigten, für ein technisches Verfahren unbefriedigend, da

- die auskristallisierte Menge zu gering ist,
- viel Bernsteinsäure mit auskristallisiert,
- es zu starken Anbackungen an Gefäßwandungen und Kühlschlangen kommt,
- die Kristallisationsgeschwindigkeit zu gering ist, weil die Lösung auch nach ca. 3 Stunden oftmals noch stark übersättigt bleibt.

Gemäß US-Patent 4 254 283 wird das gleichzeitige Auskristallisieren von Bernsteinsäure in Kauf genommen und das resultierende Gemisch aus Adipinsäure und Bernsteinsäure destillativ aufgetrennt. Der Zusatz von Schwefelsäure und Wasser zum Dicarbonsäuregemisch und die Gewinnung von Adipinsäure mittels Kristallisation sind ebenfalls beschrieben ("Separating and Recovering Adipic Acid", Japanese Kokai 54-115314).

Überraschenderweise wurde nun gefunden, daß sich die Nachteile einer Kühlkristallisation zur Rückgewinnung von Adipinsäure vermeiden lassen, wenn durch Zugabe einer wäßrigen Adipinsäurelösung mit einem Adipinsäuregehalt von 0,5 bis 6 Gew.-%, vorzugsweise einer gesättigten Lösung vor Beginn der Kristallisation die $HNO_3$-Konzentration von ca. 52 bis 60 Gew.-%, vorzugsweise 55 bis 60 Gew.-% auf ca. 35 bis 50, vorzugsweise bis ca. 45 Gew.-% erniedrigt wird, wobei gleichzeitig die Adipinsäurekonzentration konstant gehalten oder gar erhöht wird. Die genannten Prozentsätze bezüglich der $HNO_3$-Konzentration beziehen sich hier und auch nachstehend stets auf die Menge der in den Lösungen vorliegenden Salpetersäure und Wasser, ohne Berücksichtigung der organischen Bestandteile bei der Berechnung der genannten Konzentrationen. Aufgrund der erfindungsgemäßen Maßnahme kristallisiert wegen der geringen Löslichkeit in verdünnter $HNO_3$ mehr Adipinsäure aus. Der mit der zugesetzten Lösung eingeführte Adipinsäureanteil wird dadurch mehrfach überkompensiert. Die Kristallisation verläuft schneller wegen der höheren relativen Übersättigung und des Vorhandenseins von Kristallisationskeimen in der zugesetzten Adipinsäurelösung. Der Niederschlag ist gut filtrierbar. Es kristallisiert signifikant weniger Bernsteinsäure mit aus, da die Bernsteinsäurekonzentration im Gegensatz zur Adipinsäurekonzentration erniedrigt wird und da aufgrund der schnelleren Kristallisation weniger Bernsteinsäure mitgefällt wird. Gleiches gilt für die Reduzierung der Anbackungen auf den Kühlflächen. Beides ermöglicht, bei niedrigen Temperaturen zu kristallisieren und damit den Adipinsäure-Abscheidegrad zu erhöhen.

Gegenstand der Erfindung ist somit ein Verfahren zur Rückgewinnung von Adipinsäure aus bei der technischen Adipinsäureherstellung anfallenden wäßrigen Mutterlaugen mit einen Gehalt an Salpetersäure, ohne Berücksichtigung der organischen Bestandteile, berechnet als $HNO_3$ von 52 bis 60 Gew.-%, an Bernsteinsäure von 2 bis 6 Gew.-%, an Glutarsäure von 4 bis 9 Gew.-% und an Adipinsäure von 5 bis 10 Gew.-% durch selektive Kristallisation der gelösten Adipinsäure, dadurch gekennzeichnet, daß man die Mutterlauge innerhalb des Temperaturbereichs von 30 bis 60°C mit einer solchen Menge einer wäßrigen Adipinsäurelösung mit einem Adipinsäuregehalt von 0,5 bis 6 Gew.-% versetzt, daß der Gehalt des Gemischs an Salpetersäure auf 35 bis 50 Gew.-% erniedrigt wird, dann das Gemisch innerhalb eines Zeitraumes von 0,5 bis 5 Stunden um mindestens 5°C abkühlt, die hierbei auskristallisierende Adipinsäure durch Filtration isoliert und das erhaltene Filtrat der destillativen Glutarsäureaufarbeitung zuführt.

Wie bereits oben erwähnt, erfolgt technisch die Herstellung von Adipinsäure durch Oxidation technische Gemische von Cyclohexanol und Cyclohexanon mittels Salpetersäure. Aus dem hierbei anfallenden Reaktionsgemisch wird die Hauptmenge der gebildeten Adipinsäure durch Kristallisation isoliert. Die hierbei anfallende Mutterlauge enthält die Hauptmenge der als Nebenprodukte anfallenden Bernsteinsäure und Glutarsäure neben weiteren Mengen an Adipinsäure. Der zur Verhinderung der Anreicherung dieser Nebenprodukte aus dem Prozeß ausgeschleuste Teil der Mutterlauge stellt das Ausgangsmaterial für das erfindungsgemäße Verfahren dar. Diese wäßrige Lösung ist durch ein Gehalt an Salpetersäure, Bernsteinsäure, Glutarsäure und Adipinsäure in den oben genannten Konzentrationen gekennzeichnet. Zur Durchführung des erfindungsgemäßen Verfahrens wird nun diese Ausgangslösung bei einer Temperatur innerhalb des Bereichs von 30 bis 60°C mit einer wäßrigen Lösung von Adipinsäure mit einem Adipinsäuregehalt von 0,5 bis 6 Gew.-%, vorzugsweise mit einer gesättigten wäßrigen Adipinsäurelösung vermischt, wobei die Menge der zugesetzten wäßrigen Adipinsäurelösung so bemessen wird, daß der Salpetersäuregehalt des resultierenden Gemischs auf ca. 35 bis 50, vorzugsweise auf ca. 45 Gew-% absinkt. Anschließend wird das so erhaltene Gemisch während eines Zeitraums von 0,5 bis 5, vorzugsweise 1,5 Stunden um mindestens 5°C, vorzugsweise bis 20°C abgekühlt. Die hierbei auskristallisierende in einer Reinheit von mindestens 90 Gew.-% auskristallisiernde Adipinsäure wird durch Filtration isoliert und die verbleibende Lösung der Glutarsäureaufarbeitung zugeführt.

Der bei der destillativen Glutarsäureaufarbeitung anfallende Destillationsrückstand besteht ca. zu 70 bis 80 Gew.-% aus Adipinsäure und kann zur Herstellung der wäßrigen Lösung verwendet werden, die beim erfindungsgemäßen Verfahren mit der Ausgangslösung vermischt wird.

In den nachfolgenden Beispielen beziehen sich alle Prozentangaben auf Gew.-%.

Beispiele

In den nachstehenden Tabellen 1 und 2 werden die Beispiele 1 bis 5 zusammengefaßt.

Beispiel 1 ist ein Blindversuch; auch nach 3 Stunden kristallisiert Adipinsäure aus der übersättigten Lösung nicht siginfikant aus.

Beispiel 2 ist ein Vergleichsversuch, bei welchem die eingesetzte Mutterlauge ohne erfindungswesentlichen Zusatz einer wäßrige Adipinsäurelösung innerhalb von 3 Stunden um 7°C abgekühlt wird. Die abgekühlte Lösung bleibt übersättigt bezüglich Adipinsäure, die in geringem Umfang auskristallisierte Adipinsäure ist mit ca. 10 % Bernsteinsäure verunreingt.

Beispiel 3 zeigt, daß eine Erniedrigung der Salpetersäurekonzentration bereits ohne Abkühlung zu einer merklichen Erhöhung der Menge der kristallisierenden Adipinsäure führt.

Beispiel 4 und 5 sind erfindungsgemäße Beispiele, die zeigen, daß durch die erfindungsgemäße Maßnahmen eine beträchtliche Menge der in der Mutterlauge gelöst vorgelegenen Adipinsäure auskristallisiert werden kann, ohne daß sprunghaft mehr Bernsteinsäure mitgefällt wird.

## Tabelle 1

| | | | | | | | | | | |
|---|---|---|---|---|---|---|---|---|---|---|
| **VERSUCHSPARAMETER** | | | | | | | | | | |
| **Beispiel** | **Menge Mutterlauge** | | **Verdünnung** | | **Zusammensetzung Lösung** | | **$\Sigma$ AS** | **Start-** | **End-** | **Abkühl-** |
| | abs [g] | AS gel. [g] | abs [kg] | AS gel. [kg] | % $HNO_3$ org. frei | % AS In Lsg. | [kg] | temp. [°C] | temp. [°C] | zeit [h] |
| 1 | 4000 | 228,5 | - | - | 55,3 | 5,7 | 228,5 | 32 | 31 | 3 |
| 2 | 4000 | 228,5 | - | - | 55,3 | 5,7 | 228,5 | 32 | 25 | 3 |
| 3 | 4000 | 228,5 | 515 | 15,6 | 48,2 | 5,4 | 244,1 | 32 | 31 | 3 |
| 4 | 4000 | 228,5 | 515 | 15,6 | 48,2 | 5,4 | 244,1 | 32 | 25 | 3 |
| 5 | 4000 | 228,5 | 515 | 15,6 | 48,2 | 5,4 | 244,1 | 32 | 20 | 3 |

Abkürzungen: siehe Tabelle 2

EP 0 502 384 B1

Tabelle 2

| ERGEBNISSE | | | | | | | | |
|---|---|---|---|---|---|---|---|---|
| | Filtrat | | | Kristalle | | | | Σ AS | AS wdgew. |
| Beispiel | Menge [kg] | % AS in Lsg. | AS abs [kg] | Menge [kg] | Zusammensetzung % BS | % GS | % AS | [kg] | [kg] |
| 1 | 3990 | 5,4 | 218,5 | 10 | 0 | 0 | 100 | 10 | 10 |
| 2 | 3907 | 3,8 | 149,4 | 89,6 | 9,3 | 0,5 | 90,2 | 80 | 80 |
| 3 | 4398 | 3,1 | 138,4 | 107,6 | 1,3 | 0,9 | 97,9 | 105,5 | 90 |
| 4 | 4368 | 2,1 | 91,7 | 157,0 | 2,2 | 0,9 | 96,8 | 152,3 | 136,7 |
| 5 | 4342 | 1,8 | 78,5 | 174,7 | 4,2 | 0,9 | 94,9 | 166,1 | 150,5 |

Abkürzungen: AS = Adipinsäure, BS = Bernsteinsäure, GS = Glutarsäure, ASgel. = gelöste Adipinsäure, % HNO₃ org. frei = Konzentration an Salpetersäure, bezogen auf HNO₃ und Wasser, ohne Einbeziehung der organischen Bestandteile.

Σ AS = Gesamtmenge AS, AS wdgew. = wiedergewonnene Adipinsäure entsprechend der Differenz aus der Gesamtmenge Adipinsäure und der in Form der Verdünnungslösung zugesetzten Adipinsäure.

Patentansprüche

1. Verfahren zur Rückgewinnung von Adipinsäure aus bei der technischen Adipinsäureherstellung anfallenden wäßrigen Mutterlaugen mit einen Gehalt an Salpetersäure, ohne Berücksichtigung der organischen Bestandteile, berechnet als HNO₃ von 52 bis 60 Gew.-%, an Bernsteinsäure von 2 bis 6 Gew.-%, an Glutarsäure von 4 bis 9 Gew.-% und Adipinsäure von 5 bis 10 Gew.-% durch selektive Kristallisation der gelösten Adipinsäure, dadurch gekennzeichnet, daß man die Mutterlauge innerhalb des Temperaturbereichs von 30 bis 60 °C mit einer solchen Menge einer wäßrigen Adipinsäurelösung mit einem Adipinsäuregehalt von 0,5 bis 6 Gew.-% versetzt, daß der Gehalt des Gemischs an Salpetersäure auf 35 bis 50 Gew.-% erniedrigt wird, dann das Gemisch innerhalb eines Zeitraumes von 0,5 bis 5 Stunden um mindestens 5°C abkühlt, die hierbei auskristallisierende Adipinsäure durch Filtration isoliert und das erhaltene Filtrat der destillativen Glutarsäureaufarbeitung zuführt.

**2.** Verfahren gemäß Anspruch 1, dadurch gekennzeichnet, daß man zur Herstellung der mit der Mutterlauge zu vermischenden wäßrigen Adipinsäurelösungen den bei der destillativen Glutarsäureaufarbeitung anfallenden, im wesentlichen aus Adipinsäure bestehenden Destillationsrückstand verwendet.

**Claims**

**1.** A process for recovering adipic acid from aqueous mother liquors produced during the industrial manufacture of adipic acid which contain a concentration of 52 to 60 wt.% of nitric acid without taking the organic components into account, calculated as $HNO_3$, 2 to 6 wt.% of succinic acid, 4 to 9 wt.% of glutaric acid and 5 to 10 wt.% of adipic acid, by selective crystallisation of the dissolved adipic acid, characterised in that an amount of an aqueous solution of adipic acid with an adipic acid content of 0.5 to 6 wt.% sufficient to lower the concentration of nitric acid in the mixture to 35 to 50 wt.% is added to the mother liquor within the temperature range 30 to 60 °C, then the mixture is cooled by at least 5 °C over a period of 0.5 to 5 hours, the adipic acid which crystallises out being isolated by filtration and the filtrate obtained being fed to a procedure for working-up glutaric acid by distillation.

**2.** A process according to Claim 1, characterised in that the aqueous adipic acid solutions to be mixed with the mother liquor are prepared from the distillation residues produced during the working-up of glutaric acid by distillation, which essentially consist of adipic acid.

**Revendications**

**1.** Procédé pour récupérer l'acide adipique dans les liqueurs mères aqueuses produites lors de la fabrication technique de l'acide adipique, contenant une quantité d'acide nitrique, sans tenir compte des composants organiques, calculée comme $HNO_3$, de 52 à 60% en poids, d'acide succinique de 2 à 6% en poids, d'acide glutarique de 4 à 9% en poids, et d'acide adipique de 5 à 10% en poids, par cristallisation sélective de l'acide adipique dissous, procédé caractérisé en ce que l'on ajoute à la liqueur mère, dans une gamme de températures de 30 à 60 °C, une solution aqueuse d'acide adipique avec une teneur de 0,5 à 6% en poids d'acide adipique en quantité telle que la teneur du mélange en acide nitrique est abaissée à une valeur de 35 à 50% en poids, après quoi on refroidit le mélange d'au moins 5 °C en un temps de 0,5 à 5 heures, on isole par filtration l'acide adipique cristallisé et on amène le filtrat au traitement de l'acide glutarique par distillation.

**2.** Procédé selon la revendication 1, caractérisé en ce que l'on utilise le résidu de distillation, composé essentiellement d'acide adipique provenant du traitement de l'acide glutarique par distillation, pour fabriquer les solutions aqueuses d'acide adipique à mélanger avec la liqueur mère.